Europäisches Patentamt

European Patent Office

Office européen des brevets

(19)

(11) Publication number: **0 483 273 B1**

(12) **EUROPEAN PATENT SPECIFICATION**

(45) Date of publication of patent specification: **25.10.95**  (51) Int. Cl.6: **A61K 9/22**, A61K 31/71

(21) Application number: **90911939.8**

(22) Date of filing: **16.07.90**

(86) International application number:
**PCT/US90/03981**

(87) International publication number:
**WO 91/01131 (07.02.91 91/04)**

The file contains technical information submitted
after the application was filed and not included in
this specification

(54) **DISPENSER COMPRISING IONOPHORE.**

(30) Priority: **18.07.89 US 381423**

(43) Date of publication of application:
**06.05.92 Bulletin  92/19**

(45) Publication of the grant of the patent:
**25.10.95 Bulletin  95/43**

(84) Designated Contracting States:
**AT BE CH DE DK ES FR GB IT LI LU NL SE**

(56) References cited:
**EP-A- 0 025 699**
**EP-A- 0 164 241**
**WO-A-82/00094**
**GB-A- 2 179 252**

(73) Proprietor: **ALZA CORPORATION**
**950 Page Mill Road**
**P.O. Box 10950**
**Palo Alto**
**California 94303-0802 (US)**

(72) Inventor: **AYER, Atul, Devdatt**
**931 Bautista Court**
**Palo Alto, CA 94303 (US)**
Inventor: **BURKOTH, Terry, L.**
**711 Torreya Court**
**Palo Alto, CA 94303 (US)**
Inventor: **KUCZYNSKI, Anthony, L.**
**3875 Park Boulevard, 17**
**Palo Alto, CA 94306 (US)**
Inventor: **DETERS, Joseph, C.**
**979 Crooked Creek Drive**
**Los Altos, Ca 94022 (US)**

(74) Representative: **Evans, David Charles et al**
**F.J. CLEVELAND & COMPANY**
**40-43, Chancery Lane**
**London, WC2A 1JO (GB)**

Note: Within nine months from the publication of the mention of the grant of the European patent, any person
may give notice to the European Patent Office of opposition to the European patent granted. Notice of opposition
shall be filed in a written reasoned statement. It shall not be deemed to have been filed until the opposition fee
has been paid (Art. 99(1) European patent convention).

**Description**

This invention pertains to a dispenser comprising an ionophore. More particularly, the invention concerns a dispenser for the controlled administration of an ionophore to an animal for treating an infectious disease, for improving feed efficiency, and for the enhancement of growth of the animal.

Ionophores, or ion-bearers, as reported in Ann. N.Y. Acad. Sci., Vol. 264, pp 373-86, (1975), are polyether antibiotics that modulate the physiological transport of ions across biological membranes, and alter the characteristics of fermentation in the animal resulting in favorable metabolic changes. These valuable properties of ionophores led to their use as feed additives by the livestock industry. For example, the ionophores, when fed to ruminants, resulted in an improved feed-gain ratio, as reported in Feedstuffs, pp 14, 15 and 22, (1989). In one accepted use, ionophores are fed to feedlot cattle in confinement for improved feed efficiency. In this use the ionophore first is mixed with a finely ground nonmedicated feedstuff to produce a premix, which premix is added to an air-dry feed for feeding to cattle, including steers and heifers.

While the above described prior art use of ionophores results in improved feed efficiency, usually of from 5 to 8 percent, or higher, for steers and heifers, as reported in Feedstuffs, supra, serious shortcomings accompany this use. For example, since the ionophore is mixed with feed, one shortcoming is the difficulty to ascertain the amount of ionophore ingested by the animal because of feedlot losses such as spillage and scatter. Another shortcoming resides in the absence of controlled administration of known amounts of the ionophore over time, as the composition of the feed charged with the ionophore can vary with feed millers. Also, ionophores are sensitive to moisture in the environment, which moisture can adversely affect their usefulness, and the handling and transport of feeds containing ionophores can result in the segregation of particles carrying ionophores and change the concentration level to which cattle are exposed when fed over time. Then, since ionophores usually are mixed with feeds daily, this requires extra labor that adds to the cost of the ionophore-feedstuff.

EP 25,699 discloses a device for the delivery of a drug to ruminants. The device is a metal cylinder open at both ends and having the drug formulation within the cylinder. The delivery of the formulation into the ruminal environment is dependent on diffusion or erosion of the formulation at the open ends of the device; there is no means for osmotic pumping to deliver the drug. EP 164,241 discloses a ruminal drug delivery device which is a metal tube containing a core of drug and having at least one open end. The delivery of the drug from this device into the ruminal environment is dependent on diffusion or erosion of the drug formulation at the open end(s) of the device. Again, there is no means for osmotic pumping to deliver the drug. WO 82/00094 discloses a cylindrical ruminal device for delivery of a drug. The cylinder is open at one end and has a spring at the opposite end for assisting in delivering the drug from the device. The device also has extensible wings which open upon entering the rumen to provide retention in the rumen. GB-A-2,179,252 discloses an osmotic dispenser for delivering a beneficial agent in a rumen of a ruminant animal; the dispenser has a weight density of at least 1.0 to maintain it in use in the rumen. The beneficial agent may, in some embodiments, be an ionophore; the agent may be blended with a hydrophilic polymer.

None of these references addresses the problem of uncontrolled rate of delivery of an ionophore formulation into the ruminal environment or that the uncontrolled rate is caused by excessive mechanical agitation-dependent release as a result of an exit port of incorrect size.

In the light of the above presentation, it will be appreciated by those versed in the dispensing art to which this invention pertains, that a pressing need exists for a dosage form that can deliver a valuable ionophore to a biological environment of use comprising livestock for both improved feed efficiency and the enhancement of growth of the livestock. The pressing need exists also for a dosage form that can store and deliver an ionophore at a controlled rate in a substantially constant dose per unit time over a prolonged period of time essentially independent of the environment of use, which environment of use pertains to livestock that are confined and to livestock in the pasture. It will be appreciated further by those versed in the dispensing art, that if such a novel and unique dosage form is provided that can administer an ionophore in a rate controlled dose over time and, simultaneously, provide the beneficial effects, the dosage form would represent an advancement and valuable contribution in the ionophore dosage form art.

According to one aspect of the present invention therefore there is provided a dispensing device for delivering a beneficial ionophore to a fluid environment of use; the dispensing device comprising:-

(a) wall means for surrounding and forming an internal compartment, said wall means being in at least a part semipermeable, being permeable to the passage of a fluid present in the environment of use;

(b) a beneficial ionophore formulation in the compartment, said beneficial ionophore formulation forming with fluid that enters the compartment through the wall means in service a dispensable formulation;

2

(c) means in the compartment for absorbing fluid from the environment of use through the wall means by osmosis and occupying an increasing amount of space in the compartment;

(d) means in the compartment connecting the exterior of the device with the compartment for delivering the beneficial ionophore formulation to the environment of use over time;

characterised in that said connecting means is a wide mouth passageway in the wall means having a surface area selected so as to maximise the release of the beneficial ionophore formulation by osmotic pumping and to minimise the release of the ionophore by diffusion, thereby to reduce the mechanical agitation dependant release to provide delivery of ionophore in a substantially controlled rate dose.

In another aspect of the present invention there is provided a dispensing device for delivering a beneficial ionophore to a fluid environment of use; the dispensing device comprising:-

(a) wall means for surrounding and forming an internal compartment, said wall means being in at least a part semipermeable, being permeable to the passage of a fluid present in the environment of use;

(b) a beneficial ionophore formulation in the compartment, said beneficial ionophore formulation forming with fluid that enters the compartment through the wall means in service a dispensable formulation;

(c) means in the compartment for absorbing fluid from the environment of use through the wall means by osmosis and occupying an increasing amount of space in the compartment;

(d) means in the compartment connecting the exterior of the device with the compartment for delivering the beneficial ionophore formulation to the environment of use over time;

characterised in that the release rate of ionophore from the device is defined by the expression:-

$$[dm/dt]_t = [dm/dt]_o + [dm/dt]_d$$

in which $[dm/dt]_t$ is the total release rate of the ionophore, $[dm/dt]_o$ is the release rate of the beneficial agent due to the action of the osmotic pumping and $[dm/dt]_d$ is the release rate of the beneficial ionophore due to diffusion and in which $[dm/dt]_d$ is not greater than 33% of $[dm/dt]_t$.

Another object of the present invention is to provide a dosage form for administering an ionophore in a rate controlled dose over a prolonged period of time to livestock for the improvement of feed efficiency and the enhancement of growth.

Another object of the present invention is to provide a dosage form for administering an ionophore in a lower overall dosage than the dose required if mixed with feedstuffs.

Another object of the present invention is to provide a dosage form comprising an ionophore antibiotic for administering it to a ruminant for producing metabolic changes in the rumen.

Another object of the present invention is to provide a dosage form comprising an ionophore that modulates the transport of ions across biological cellular membranes and when administered to ruminants the ionophores improve feed-gain ratios.

Another object of the invention is to provide a novel dosage form manufactured as an osmotic dispenser that can administer an ionophore to a biological receptor site to produce the desired beneficial ionophore effects.

Another object of the present invention is to provide a dosage form that can deliver a substantially water-insoluble ionophore at a controlled rate over time.

Another object of the present invention is to provide a dosage form for administering an ionophore, which dosage form comprises a composition that occupies space in the dosage form for displacing a composition comprising an ionophore from the dosage form.

Another object of the present invention is to provide a novel composition of matter comprising an ionophore, which composition can be dispensed from a dosage form to livestock in the pasture and to livestock in confinement.

Another object of the present invention is to provide a method for administering an ionophore in a rate-controlled dose per unit time for its beneficial effect.

Another object of the invention is to provide a dispenser with high drug loading of an ionophore that is self-contained, self-starting and self-powered in a fluid environment of use.

Another object of the invention is to provide a dispenser comprising a semipermeable wall that surrounds in at least a part an internal lumen, which lumen comprises a carrier comprising an ionophore and which carrier initially occupies a major portion of the lumen except for a driving member and a densifier, which dispenser delivers the ionophore by the combined physical -chemical operations of the driving member urging the displaceable carrier through an opening in the wall to the environment of use.

Another object of the invention is to provide a dispenser for administering an ionophore in a rate-controlled dose or amount by utilizing one or more exit passageways in the wall for delivering the ionophore from the dispenser, the diameter of each exit passageway being of a size to maximize release of the ionophore by osmotic pumping and minimize release of the ionophore by diffusion to avoid mechanical agitation dependent ionophore release and to therefore provide delivery of the ionophore in a rate-controlled dose.

Another object of the present invention is to provide a dispenser comprising a pharmaceutical carrier containing an ionophore that is a dispensable composition, that is innocuous, and when upon its displacement from the dispenser substantially avoids mammalian tissue irritation and interaction with mammalian protein tissue.

Another object of the invention is to provide a composition sized, shaped and adapted for housing in a dispenser which composition comprises from 0.01 weight percent (wt%) to 95 weight percent of an ionophore and a pharmaceutical carrier for the ionophore with the total amount of all ingredients in the composition equal to 100 weight percent.

Other objects, features and advantages of the invention will be more apparent to those versed in the dispensing and veterinary arts from the following detailed specification, taken in conjunction with the drawings and the accompanying claims.

Typical dimensions of the wide mouth passageway in the dispenser of this invention are between 50 mil (1.27 mm) and 400 mil (10.2 mm).

Following is a description by way of example only and with reference to the accompanying drawings of methods of carrying the present invention in to effect.

Figure 1 is a view of a dosage form designed and manufactured as a dispenser for administering a beneficial ionophore to a warm-blooded animal;

Figure 2 is a view of another dosage form provided by the invention sized and adapted for administering a beneficial ionophore to a warm-blooded animal over a prolonged period of time;

Figure 3 is an opened view of the dosage form of Figure 1 through 3-3 thereof for illustrating the structure of the dosage form;

Figure 4 is an opened view of the dosage form of Figure 2 through 5-5 thereof for illustrating a different structural embodiment of the dosage form;

Figure 5 is an opened view of the dosage form of Figure 1, wherein the dosage form depicted in Figure 5 comprises a different internal arrangement and exit means for delivering an ionophore from the dosage form;

Figure 6 is an opened view of the dosage form of Figure 1, wherein the dosage form in Figure 6 illustrates another embodiment of the internal members and the exit means; and,

Figures 7 through 11 depict release rate patterns for dispensers provided by the invention.

In the drawing figures and in the specification, like parts in related figures are identified by like reference numerals. The terms appearing earlier in the specification, and in the description of the drawing figures, as well as embodiments thereof, are further detailed elsewhere in the disclosure.

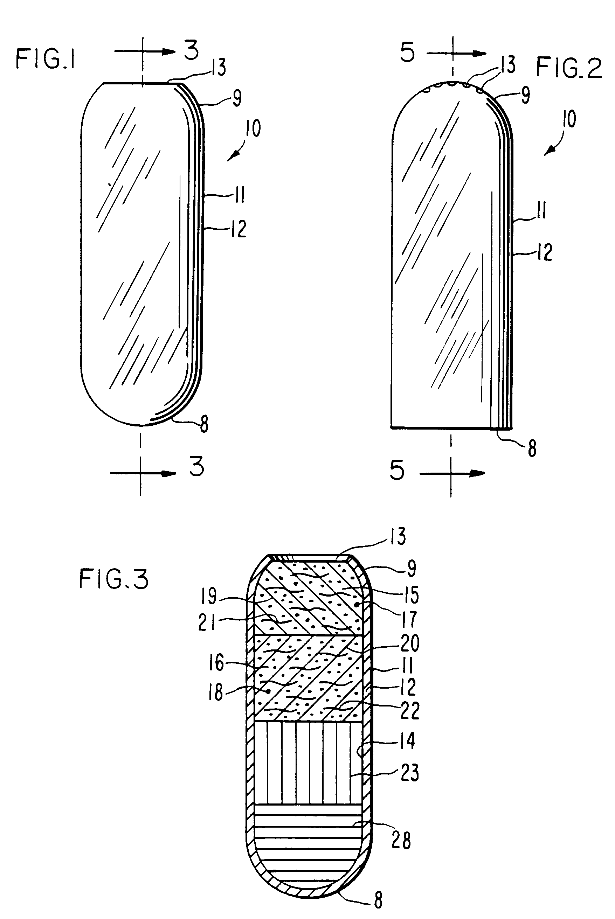

In Figure 1, a dosage form 10 is seen comprising a body member 11 comprising a wall 12 that surrounds an internal lumen not seen in Figure 1. Dosage form 10 comprises a lead end 9 and a rear end 8. Lead end 9 comprises a wide exit passageway 13 for releasing a beneficial ionophore from dosage form 10 to a biological environment of use.

Figure 2 illustrates another embodiment of dosage form 10 provided by this invention. In Figure 2, dosage form 10 comprises lead end 9, rear end 8, body 11 and wall 12. Lead end 9 comprises more than one, or a multiplicity of exit passageways 13 through wall 12 for releasing a beneficial ionophore from dosage form 10.

In Figure 3, dosage form 10 of Figure 1, is seen in opened section through 3-3 of Figure 1. In Figure 3, dosage form 10 comprises lead end 9, rear end 8, a body 11, and a wall 12 that surrounds and forms an internal compartment 14 that communicates through a wide exit passageway 13 with the exterior of dosage form 10. Wall 12, of dosage form 10, comprises totally a semipermeable composition, or wall 12 comprises at least in part a semipermeable composition. The remainder of wall 12, in the latter embodiment, may comprise a composition that is substantially impermeable to the passage of an exterior fluid present in the environment of use, and it is substantially impermeable to the passage of ingredients present inside dosage form 10. Both semipermeable or non-permeable portions of wall 12 are non-toxic and maintains its physical and chemical integrity during the delivery of the beneficial ionophore from dosage form 10.

Internal composition or lumen 14 comprises a first composition 15 and a second composition 16. The first composition 15 comprises a beneficial ionophore represented by dots 17, and the second composition 16 comprises a beneficial ionophore represented by dots 18. The first and second compositions comprise at least one or more than one ionophore. The first and second compositions comprise like or unlike ionophores. The first and second compositions comprise the same dosage unit amounts or the compositions comprise different dosage unit amounts of an ionophore. First composition 15 also comprises a pharmaceutically acceptable carrier represented by slanted lines 19 for ionophore 17, and the second composition 16 comprises a pharmaceutically acceptable carrier represented by slanted lines 20 for ionophore 18. Carriers 19 and 20 can be the same or different in compositions 15 and 16. In both embodiments, carriers 19 and 20 imbibe and/or absorb an external fluid that enters compartment 14 and form thereby a dispensable composition for transporting ionophores 17 and 18 from dosage form 10. First composition 15 and second composition 16 in a preferred optional embodiment comprise a binder, a tableting agent or a lubricant represented in composition 15 by wavy line 21 and in composition 16 by wavy line 22. Composition forming members 21 and 22 can be the same or they can be different in compositions 15 and 16.

Dosage form 10 in compartment 14 further comprises an expandable driving member 23 that is in contact with second composition 16. Expandable driving member 23 has a shape that corresponds to the internal shape of compartment 14. Expandable driving member 23, in the presence of an external fluid that enters compartment 14, imbibes and/or absorbs the fluid, increases in size, and thereby pushes against composition 16 to displace first composition 15 and second composition 16 from dosage form 10. Compartment 14 also comprises a dense member 24 or densifier that is in contact with expandable member 23. Dense member 24 is an important component of dosage form 10 for keeping dosage form 10 in the rumen of an animal over a prolonged period of time.

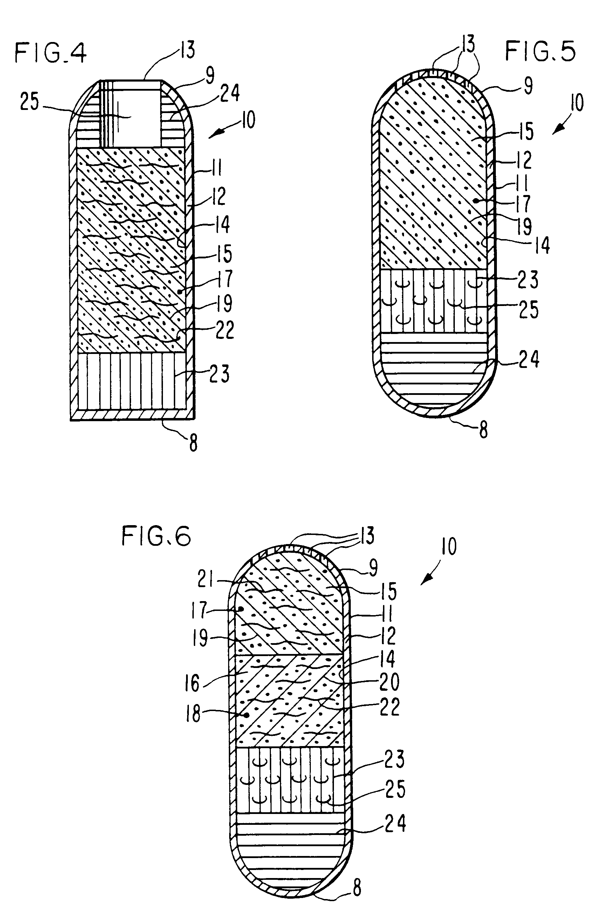

Drawing Figure 4 depicts another manufacture provided by the invention. In drawing Figure 4, dosage form 10 comprises a body 11, and a wall 12 that surrounds and defines an internal compartment 14. Wall 12 comprises in a presently preferred embodiment a semipermeable composition that is substantially permeable to the passage of an external fluid, and it is substantially impermeable to the passage of ingredients contained in dosage form 10. Wall 12 is non-toxic and it keeps its physical and chemical integrity, that is, wall 12 doesn't erode during the dispensing period. Dosage form 10 also comprises a single composition 15. Composition 15 comprises at least one ionophore 17 homogeneously or heterogeneously dispensed in a pharmaceutically acceptable carrier 19. Carrier 19 is substantially dry during storage of dosage form 10, and when dosage form 10 is in operation in a fluid environment of use and carrier 19 is in contact with the fluid, carrier 19 changes from a rested state to a dispensable state form delivering ionophore 17 from dosage form 10. Dosage form 10 also comprises a dense member 24 positioned next to a wide-mouth passageway 13 in wall 12. Dense member 24 has a shape that corresponds to the shape of lead end 9 and to the inside shape of dosage form 10. A passageway 25 extends through dense member 24 for delivering beneficial composition 15 comprising ionophore 17 through dense member 24 and then through passageway 13 from dosage form 10. Compartment 14 also comprises an expandable member distant from passageway 13 at rear end 8. Expandable member 23 is in contact with composition 15 for displacing composition 15 through passageways 25 and 13 from dosage form 10. Composition 15 optionally comprises a binder, a tableting aid or a lubricant 22 for enhancing the manufacture and the displacement of composition 15 from dosage form 10.

Drawing Figure 5 depicts, in opened view, another manufacture provided by the invention. In drawing Figure 5, dosage form 10 comprises a body 11 and a wall 12 that surrounds and forms internal compartment 14. Internal compartment 14 comprises composition 15 which composition 15 comprises a pharmaceutically acceptable carrier 19 containing ionophore 17. Compartment 14 also comprises expandable member 23, which member 23 optionally comprises an osmotically effective solute 25. A densifier 24 is present in dosage form 10 position distant from lead end 9. Dosage form 10 comprises a multiplicity of passageways 13 in wall 12 at lead end 9. Passageways 13 comprise a number of smaller openings, generally in a shower-head or screen-like arrangement. The arrangement breaks-up composition 15 when emerging through opening 13.

Figure 6 illustrates another embodiment of dosage form 10 provided by the invention. In Figure 6, dosage form 10 is seen in opened section and it comprises rear end 8, leading end 9, body 11, openings 13, lumen 14, first composition 15, second composition 16, ionophore 17, ionophore 18, nontoxic carrier 19, nontoxic carrier 20, tableting aids 21, tableting aids 22, expandable member 23, densifier 24, and osmotically effective solute 25. Dosage form 10 comprises a multiplicity of exit openings 13 that are essentially means for breaking-up composition 15 and composition 16 as they are pushed at a controlled rate through the openings in wall 12. Exit means 13 also function to prevent a premature ejection of a composition from dosage form 10.

The dosage form of the invention can be sized and shaped for delivering an ionophore to a variety of animals. For example, the dosage form can be adapted for delivering an ionophore to ruminant animals including cattle, sheep, giraffe, deer, goat, bison and camels, and more particularly cattle and sheep, that comprise an important group of animals that require periodic administration of an ionophore. Dosage form 10 can embrace a capsule-like shape and in one design have a diameter of about 0.5 inches to 1 inch (1.3 cm to 2.5 cm) and a length of about 0.5 inches to 2.5 inches (1.3 cm to 6.6 cm). For use with cattle, dosage form 10 has a diameter of about 0.5 inches to 1.5 inches (1.3 cm to 3.8 cm), and a length of about 1 inch to 4 inches (2.5 cm to 10.2 cm).

While Figures 1 through 6 illustrate various dosage forms that can be made according to the invention, it is to be understood these dosage forms are not to be construed as limiting the invention as the dosage form can take other shapes, sizes and forms for delivering a beneficial ionophore to the biological environment of use. The dosage form can be used in veterinary clinics, farms, zoos, laboratories, on the range, in feed lots, and other environments of use.

In accordance with the practice of this invention it has now been found that wall 12 can be made with a wall forming composition that does not adversely affect the animal, and it does not adversely affect the beneficial ionophore and other ingredients in dosage form 10. Wall 12 is semipermeable, that is, the wall is permeable to the passage of an external fluid such as water and biological fluids, and it is substantially impermeable to the passage of ionophore. Typical materials used for forming wall 12 are, in one embodiment, cellulose esters, cellulose ethers, and cellulose ester-ethers. The cellulose polymers have a degree of substitution, D.S., on their anhydroglucose unit of from greater than 0 up to 3 inclusive. By degree of substitution is meant the average number of hydroxyl groups originally present on the anhydroglucose unit comprising the cellulose polymer that are replaced by a substituting group. Representative materials include a member selected from the group consisting of a cellulose acylate, cellulose diacylate, cellulose triacylate; cellulose acetate, cellulose diacetate, cellulose triacetate; mono-, di-, and tricellulose alkanylates; mono-, di-, and tricellulose aroylates, and the like. Exemplary polymers include cellulose acetate having a D.S. up to 1 and an acetyl content up to 21%; cellulose acetate having a D.S. of 1.8 to 2.3 and an acetyl content of 32% to 39%; cellulose diacetate having a D.S. of 1 to 2 and an acetyl content of 21% to 35%; cellulose triacetate having a D.S. of 2 to 3 and am acetyl content of 34% to 44.8%, and the like. More specific cellulose polymers include cellulose propionate having a D.S. of 1.8, a propyl content of 39.2% to 45% and a hydroxyl content of 2.8% to 5.4%; cellulose acetate butyrate having a D.S. of 1.8, an acetyl content of 13% to 15% and a butyryl content of 34% to 39%; cellulose acetate butyrate having an acetyl content of 2% to 29%, a butyryl content of 17% to 53% and a hydroxyl content of 0.5% to 4.7%; cellulose triacylate having a D.S. of 2.9 to 3 such as cellulose trivalerate, cellulsoe trilaurate, cellulose tripalmitate, cellulose trisuccinate and cellulose trioctanoate; cellulose diacylate having a D.S. of 2.2 to 2.6 such as cellulose disuccinate, cellulose dipalmitate, cellulose dioctanoate, cellulose dipentanoate; coesters of cellulose such as cellulose acetate butyrate, cellulose acetate propionate, and the like.

Additional polymers include ethyl cellulose of various degrees of etherification with ethoxy content of from 40% to 55%; cellulose acetate ethyl carbamate; cellulose acetate methyl carbamate; cellulose acetate diethyl aminoacetate; semipermeable polyurethanes; semipermeable sulfonated polystyrenes; semipermeable cross-linked polymers formed by the coprecipitation of a polyanion and a polycation as disclosed in U.S. Pat. Nos. 3,173,876; 3,276,586; 3,541,005; 3,541,006; 3,546,142; 4,595,583; 4,783,337; and the like. Semipermeable polymers also are disclosed by Loeb and Sourirajan in U.S. Pat. No. 3,133,132. Semipermeable lightly cross-linked polymers, semipermeable cross-linked poly(sodium styrene sulfonate), semipermeable cross-linked poly(vinylbenzyltrimethyl) ammonium chloride; semipermeable polymers exhibiting a fluid permeability of $2.5 \times 10^{-8}$ to $2.5 \times 10^{-4}$ ($cm^2/hr$ . atm) expressed per atmosphere of hydrostatic or osmotic pressure difference across a semipermeable membrane are disclosed in U.S. Pat. No. 3,845,770; 3,916,899 and 4,160,020; and in Handbook of Common Polymers by Scott, J.R. and Roff W.J., (1971) published by CRC Press Cleveland, OH.

Semipermeable wall 12 also can comprise a flux regulating agent. The flux regulating agent is a compound that assists in regulating the permeability of a fluid through the semipermeable wall. Flux regulating agents that increase the permeability of a wall to fluid, such as water, are essentially hydrophilic. The amount of regulator in the wall, when incorporated therein, generally is from about 0.01 weight percent, (wt%), to 35 wt%, or more. The flux regulator agents in one embodiment comprise a member selected from the group consisting of a polyhydric alcohol, polyalkylene glycol, polyalkylenediol, polyester of alkylene glycol, and the like. Typical flux enhancers comprise polyethylene glycol 300, 400, 600, 1500, 4000, 6000 and the like; low molecular weight glycols such as polypropylene glycol, polybutylene glycol, and polyamylene glycol; the polyalkylenediols such as poly(1,3-propanediol), poly(1,4-butanediol),poly(1,6-hexanediol) and the like; aliphatic diols such as 1,3-butylene glycol, 1,4-pentamethylene glycol, 1-4,hexamethylene glycol, and the like; alkylene triols such as glycerine, 1,2,3-butanetriol, 1,2,4-hexanetriol, 1,3,6-hexanetriol, and the like; esters such as ethylene glycol diproprionate, ethylene glycol butyrate, butylene glycol dipropionate; and the like.

Semipermeable wall 12 optionally comprises a plasticizer, for imparting flexibility and elongation properties to the wall, for making the wall less to nonbrittle, and for enhancing the manufacturing properties of the wall. Plasticizers useful for the present purpose comprise dihexyl phthalate, butyl octyl phthalate, triacetin, dioctyl azelate, epoxidized tallate, sucrose acetate isobutyrate, epoxidized soybean oil, phosphate esters, tricresyl phosphate, triacetyl phosphate, adipate esters, sebacate esters, and other nontoxic plasticizers. The amount of plasticizer in wall 12 when incorporated therein, is about 0.01 wt% to 40 wt%, or more.

Representative of beneficial ionophores that can be dispensed using the dosage form of this invention comprise natural and synthetic ionophores. The ionophores are polyethers and they possess the ability to transport mono and divalent cations across lipid bilayers, which lie within biological membranes. The ionophores possess unique properties which derive from their ability to perturb transmembrane ion gradients and electrical potentials. The ability of ionophores to complex and transport ions leads to their applications as antibiotics against gram-positive microorganisms, against mycobacteria, as growth promotants in ruminants such as cattle and sheep, and for improved feed utilization as seen by increasing the efficiency of meat production. Ionophores that can be stored and dispensed by the dosage form of this invention comprise a member selected from the group consisting of azolomycin, valinomycin, enniactin, monactin, nonactin, dinactin, trinactin, virginamycin, tetronasin, semduramicin, monensin, monensin sodium, monensin factor B, monensin factor C, nigericin, narasin also known as methyl salinomycin, salinomycin, isolasalocid, lasalocid, lysocellin, septamycin, laidomycin, lonomycin, lenotemycin, grisorixin, ferensimycin, alborixin, rosgramicin, ethermoycin, sodium lysocellin, and the like. The ionophores also comprise the pharmaceutically acceptable derivatives having ionophore activities, such as the pharmaceutically acceptable salts, the alkylated and alkenyl derivatives, the monoglycoside and diglycoside derivatives, the hydroxylated derivatives, the free acid, the hydrate, and the like. In one presently preferred embodiment, the ionophores exhibit a molecular weight of about 350 to 2500. The presently preferred amount of an ionophore in a dosage form, present in a single composition, or in a first and second composition, generally is from 10 milligrams to 100 grams. The amount of ionophore in a first composition can be from 10 mg to 100 g and the amount of ionophore in a second composition can be from 10 mg to 100 g with the total amount of ionophore in both compositions equal to 100 g. The amount of ionophore in a first and in a second composition can be the same or different. The compositions can comprise one, or more than one like or unlike ionophore. The dosage form provided by the invention can deliver various dosage amounts of an ionophore, for example, from 10 mg per day to 500 mg per day for 200 days or longer. The ionophores are known in the ionophore art in Kirk-Othmer Encyclopedia, Vol. 3, pp 47-64, (1978); Ann. N.Y. Acad. Sci., Vol. 264, pp 373-86, (1975); and ACS Sym., Ser. 140, pp 1-22, (1980). The ionophore can be present as a base, as a salt, or as a derivative thereof.

The pharmaceutically acceptable carrier 19 and 20 forming the first and second compositions 15 and 16 and comprising ionophores 17 and 18 comprise pharmaceutically acceptable polymers that are hydrophilic, nontoxic, and substantially free of reaction with an ionophore and other members forming dosage form 10. The pharmaceutically acceptable carrier comprising an ionophore provides unexpected advantages comprising (a) the ability to store a high dosage amount up to 95 wt% of an ionophore, (b) the ability to dispense an ionophore in controlled, small doses over a prolonged time up to 6 months, (c) the ability to substantially protect a fluid sensitive ionophore from fluid that enters the dosage form by harboring the ionophore within its polymeric structure; and (d) the ability to charge high loadings of an ionophore in a polymer carrier that undergoes change from a rested state to a dispensable state possessing a dispensable viscosity, or to a semisolid dispensable state during operation of the dosage form. The polymer carriers useful for the present purpose comprise a member selected from the group consisting of polyethylene oxide polymers having a 200,000 to 7,500,000 molecular weight; carboxy vinyl polymers, sometimes referred to carboxypolymethylene, commercially available as Carbopol® polymer possessing a 200,000 to 5,000,000 molecular weight; poly(vinyl pyrrolidone) having a 125,000 to 460,000 molecular weight; poly-(hydroxyalkyl methacrylate) having a 100,000 to 5,000,000 molecular weight; polysaccharides such as agar, karaya, tragacanth, algin, guar, nanthan, and the like, having a 50,000 to 2,000,000 molecular weight and the like.

Expandable layer 23 useful for displacing the first and/or the second composition from the dosage form comprises a hydrogel composition. The hydrogel composition is noncross-linked or optionally lightly cross-linked and it possesses osmotic properties like the ability to imbibe an exterior fluid through the semipermeable wall and exhibit an osmotic pressure gradient across the semipermeable wall. The polymer exhibits the ability to retain a significant fraction of the imbibed fluid in the polymer structure. The polymers in a preferred embodiment are gel polymers that can swell or expand to a very high degree, usually exhibiting a 2 to 50 fold volume increase, thereby pushing and displacing the composition comprising the ionophore from the dosage form. The swellable, hydrophilic polymers also are known as osmopolymers. The polymers can be of plant, animal or synthetic origin. Polymeric materials useful for forming the expandable layer comprise anionic and cationic hydrogels; polyelectrolyte complexes; a mixture of agar and carboxymethyl cellulose; a composition comprising methyl cellulose mixed with sparingly cross-linked agar; water swellable polymer of N-vinyl lactams; polyethylene oxide possessing a 1,000,000 to 10,000,000 molecular weight; starch graft polymers; sodium carboxymethylcellulose having a 90,000 to 1,000,000 molecular weight; a composition comprising sodium carboxymethylcellulose and a member selected from the group consisting of hydroxypropylcellulose and hydroxypropylmethylcellulose, and the like. Representative polymers possessing hydrophilic properties are known in U.S. Pat. Nos. 3,865,108; 4,002,173; 4,207,893; and 4,327,725; and in Handbook of Common Polymers by Scott and Roff, published by the Cleveland Rubber Company, Cleveland, OH.

Expandable polymer layer 23 optionally comprises an osmotically effective compound. Osmotically effective compounds also are known as osmotically effective solutes and as osmagents. The osmotically effective compounds exhibit an osmotic pressure gradient across semipermeable wall 12, and they imbibe fluid into compartment 14. The presence of this imbibed fluid provides added fluid for the expandable polymer to absorb and increase its volume, and the imbibed fluid continuously fills the driving area of the compartment and forms a push member that urges the first and/or second compositions from dosage form 10. Osmotically effective compounds useful for the present purpose comprise magnesium sulfate, magnesium chloride, sodium chloride, potassium chloride, lithium chloride, potassium sulfate, sodium sulfate, mannitol, urea, sorbitol, inositol, sucrose, glucose, a mixture of sodium chloride and magnesium chloride, a mixture of potassium chloride and sucrose, and the like. The osmotic pressure in kg/cm$^2$ (atmospheres, atm) of osmotically effective compounds suitable for the invention will be greater than zero kg/cm$^2$ (zero atm), generally from about 8.3 kg/cm$^2$ (8 atm) up to about 516.7 kg/cm$^2$ (500 atm), or higher. The amount of osmotically effective compound blended homogeneously or heterogeneously with the swellable polymer is from 0.02 wt% to 50 wt%. Osmotically effective polymers are known in U.S. Pat. Nos. 4,595,583 and in 4,783,337.

Composition forming members, or tableting aids 21 and 22 used to provide compositions 15 and 16 comprise binders that impart cohesive qualities to the composition such as poly(vinyl pyrrolidone), natural and synthetic gums such as sodium alginate methylcellulose, hydroxypropylmethylcellulose, Veegum®, waxes, and the like; lubricants for enhancing the rate of flow of the tablet granulation, to prevent adhesion to dies and punches during tableting processes such as a magnesium stearate, calcium stearate, stearic acid, talc, lycopodium, and the like; coloring agents for esthetic qualities and identification such as FD&C Blue No. 1; surfactants that aid in dispensing the ionophore after its release from the dosage form, such as anionic, cationic, nonionic and amphoteric surfactants; and the like. Composition members are disclosed in Pharmaceutical Sciences, Remington, 14th Ed., (1970). The amount of composition member present in the composition is about 0.01 wt% to 20 wt%.

The dense member 24, also referred to as densifier 24, is used in delivery system 10 to retain the dosage form in the rumenreticular sac of a ruminant. Dense member 24 lets dosage form 10 remain in the rumen over a prolonged period of time, rather than letting it pass into the alimentary tract and be eliminated therefrom. As dosage form 10 remains in the rumen, beneficial ionophore is delivered at a controlled rate to the ruminant over a prolonged period up to 6 months or longer. Generally, dense member 24 will have a density of from about 1.0 to 8, or higher, with the density in a presently preferred embodiment exhibiting a specific gravity of from 1.5 to 7.6. For the ruminants cattle and sheep, it is presently preferred dense member 24 exhibit a density to assure complete system density of 2 to 3, or greater. Materials that have a density that can be used for forming dense member 24 include iron, iron shot, iron shot coated with iron oxide, iron shot magnesium alloy, steel, stainless steel, copper oxide, a mixture of copper oxide and iron powder, and the like. Dense member 24 in dosage form 10 can embrace different embodiments. For example, dense member 24 can be machined, or cast as a single, solid piece made of stainless steel having a density of 7.6. The solid member is made having a curved shape that corresponds to the internal shape of system 10. Dense member 24 in another manufacture can be a solid member having an axially aligned bore that extends through the length of the member. In another embodiment dense member 20 can comprise a plurality of dense pellets.

The expression, "passageway 13", as used herein, denotes an opening or a means in wall 12 suitable for releasing the composition comprising the ionophore from dosage form 10. The invention provides a passageway for releasing a composition intact, and it provides a passageway member for dividing the original composition into smaller compositions as it is released from dosage form 10. The release of a composition from dosage form 10, in either instance, embodies a combination of osmotic hydrodynamic pumping and diffusion properties through an exit passageway or through a series of exit passageways functioning as an exit port. The dosage form provided by this invention maximizes the release by osmotic pumping and minimizes the release by diffusion, thereby substantially avoiding mechanical agitation

8

dependent drug release. The release rate pattern from a drug dosage form, for example, designed to deliver 85 mg/day of the drug lysocellin is as follows in Equation (1):

$$\left[\frac{dm}{dt}\right]_t = \left[\frac{dm}{dt}\right]_o + \left[\frac{dm}{dt}\right]_d \qquad (1)$$

wherein;

$\left[\dfrac{dm}{dt}\right]_t$ is the total amount of drug released from the device per unit time (mg/day);

$\left[\dfrac{dm}{dt}\right]_o$ is the amount of drug released per unit time (mg/day) due to osmotic pumping; and

$\left[\dfrac{dm}{dt}\right]_d$ is the amount of drug released per unit time (mg/day) due to diffusion.

Then, assuming that negligible water migrates into the drug composition through the wall, Equation (2) follows:

$$\left[\frac{dm}{dt}\right]_o = V \cdot \rho d \cdot L = \left[\frac{k}{h}\right] Ap \cdot \Delta\pi \cdot \rho d \cdot L \qquad (2)$$

wherein;

V is the release rate from the dosage form at 40°C in cc/day;
$\rho_d$ is the drug + pharmaceutical carrier density in mg/cc;
L is the percent drug loading;
k is the water permeability of the wall at 40°C in

$$\frac{cm^3 \ mm}{cm^2 \ hr \ atm} \ (or \ in \ mil) \ ;$$

h is the wall thickness in mm (or in mil);
Ap is the surface of the push-composition in contact with the wall. The bottom and top surfaces of the push composition are in contact with density element and the drug composition respectively; and,
$\Delta\pi$ is the water imbibition pressure kg/cm$^2$ (atm).
During operation of the dosage form, Ap x $\Delta\pi$ remains constant; and their operation can be illustrated by the accompanying graph wherein Ap x $\Delta\pi$ = C; and,

Δπ                                                    Ap

Time

wherein any decrease in osmotic activity is compensated for by an increase in the area of the push composition in contact with the wall. Therefore, it follows according to Equations (3) and (4):

$$\left[\frac{dm}{dt}\right]_o = \left[\frac{k}{h}\right] C \cdot \rho_d \cdot L \qquad (3)$$

$$\left[\frac{dm}{dt}\right]_o = \left[\frac{k\pi \times Ap}{h}\right] \rho_d \cdot L \qquad (4)$$

The amount of drug delivered due to diffusion into an environment of use initially free of drug is set forth by Equation 5:

$$\left[\frac{dm}{dt}\right]_d = \left[\frac{D \; Sep \; Cs}{h}\right] L \qquad (5)$$

wherein:

$$\left[\frac{dm}{dt}\right]_d$$

is the amount of formulation, comprising lysocellin and pharmaceutical carrier diffusing through the exit port per unit time (mg/day);

D is the diffusion coefficient of the formulated drug in ruminal fluid in $cm^2$/day;

Sep is the surface area of the exit port in $cm^2$;

h is the thicknesses of the diffusion layer in cm;

Cs is the solubility of formulated drug in ruminal fluid in mg/ml;

L is the percent drug present in the formulation; and,

D/h = K is the dissolution rate constant in cm/day.

The composition comprising the ionophore drug is intermittently eroded at the exit passageway in the rumen, and the thickness of the diffusion layer varies from zero to several mm in thickness. The diffusion layer at the dosage form environment of use interrace is very thin and will have minimal effect on the amount of formulated drug diffusing through the exit passageway into the ruminal fluid. The two major factors which contribute to the diffusion through the exit passageway are:

(1) Surface area of the exit port (Sep); and,

(2) Solubility of the pharmaceutical carrier and the lysocellin ionophore in ruminal fluid (Cs).

Following the above presentation, the osmotic release rate for the dosage form comprising a lysocellin ionophore composition can be calculated to be 67 mg/day. The total release rate $(dm/dt)_t$ for the lysocellin is 85 mg/day. The diffusional release rate for lysocellin is therefore 18 mg/day.

The effect of the exit passageway diameter increase on the lysocellin release rate was calculated to give the following values (for the 85% drug loading):

| $(dm/dt)_t$ | $(dm/dt)_o$ | $(dm/dt)_d$ | Exit passageway diameter | (Sep)exit passageway surface area |
|---|---|---|---|---|
| mg/day | mg/day | mg/day | mm (mil) | cm$^2$ |
| 68.7 | 66.7 | 2.04 | 2.54 (100) | 0.051 |
| 74.8 | 66.7 | 8.13 | 5.1 (200) | 0.203 |
| 85.0 | 66.7 | 18.31 | 7.6 (300) | 0.456 |
| 85.0 | 66.7 | 18.31 | 2.5x9 (100x9) | 0.456 |
| 99.2 | 66.7 | 32.52 | 10.2 (400) | 0.810 |

The diffusional release increases with an increase in the exit diameter. For the 7.7 mm (300 mil) exit diameter the diffusional release is 21.5%, but for a 10.2 mm (400 ml) exit the diffusional release is 33% of the total lysocellin release rate.

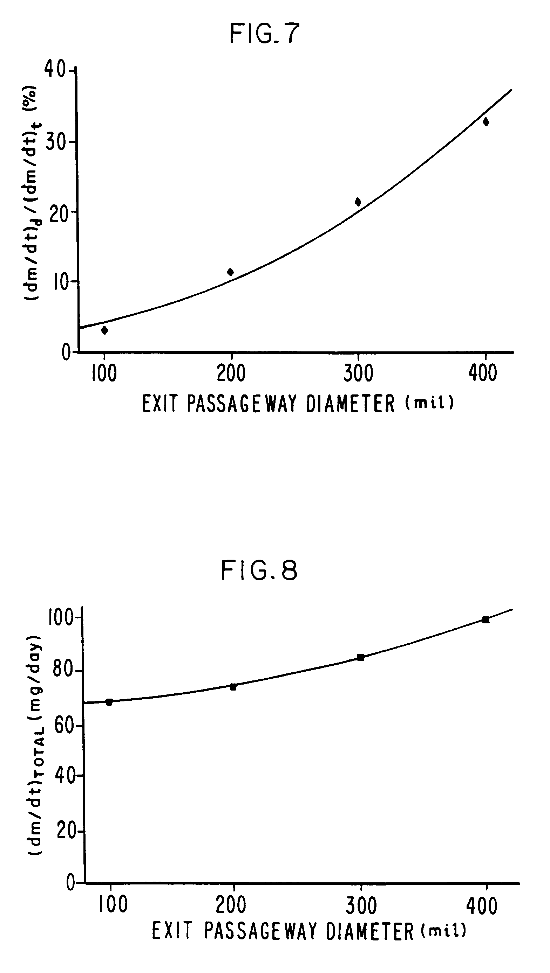

Accompanying Figure 7 shows the effects of the exit passageway diameter on the diffusional release of lysocellin. In Figure 7, one mil equals 0.0254 mm.

Accompanying Figure 8 shows the effects of the exit passageway diameter on the total release of lysocellin from the dosage form.

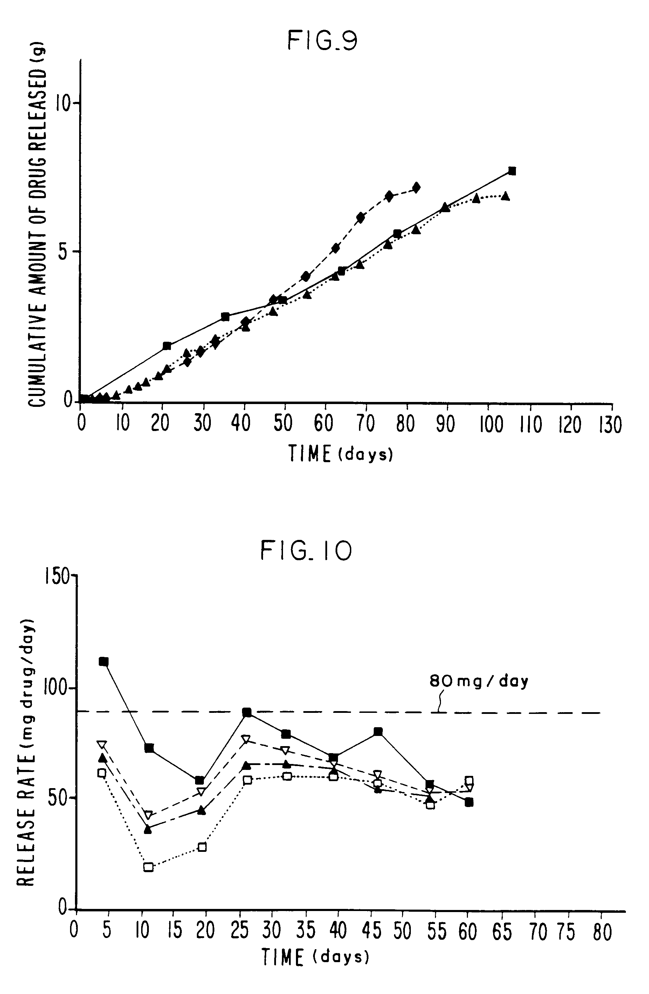

Accompanying Figure 9 depicts the functionality of the dosage form. The release rate $(dm/dt)_t$ from the dosage form is about 85 mg/day in vivo. In Figure 9, the in vivo testing in the rumen of a cow is indicated by squares, the in vitro testing in buffer at pH 8 is indicated by diamonds, and the in vitro testing in artificial ruminal fluid is indicated by triangles. The buffer was a pH 8 buffer consisting of 140.70 g of potassium phosphate, and 38.64 g of sodium hydroxide dissolved in 20 liters of distilled water. The artificial rumen fluid consists of 124.69 g of sodium acetate, 53.99 g of sodium propionate, 21.00 g of sodium bicarbonate, 81.02 g of sodium succinate, and 29.92 g of butyric acid dissolved in 20 liters of distilled water then bubbled with carbon dioxide for 10 minutes.

Accompanying Figure 10 shows the results of the effect of the exit diameter increase on the lysocellin release rate. In Figure 10, the horizontal line connected by dots indicates a constant release rate of 80 mg/day. The line with empty squares depicts the release rate through a 10.2 mm (400 ml) passageway for a composition comprising lysocellin and a hydrophilic polymer having a 7,500,000 molecular weight; the line with triangles pointed up depicts the release rate through a 7.6 mm (300 mil) passageway by a composition comprising lysocellin and a hydrophilic polymer having a 7,500,000 molecular weight; the line with the triangles pointed down depicts the release rate through a 7.6 mm (300 mil) passageway for a composition comprising lysocellin and a hydrophilic polymer having a 5,000,000 molecular weight; and, the line with filled squares depicts the release rate through a 5.1 mm (200 mil) orifice for a composition comprising lysocellin and a hydrophilic polymer having a 5,000,000 molecular weight, and the line in dashes across the figure indicates a release of 80 mg/day.

The table presented immediately below sets forth the results obtained by comparing the calculated values with the experimental values obtained for a 79% lysocellin loading in a drug composition.

| Drug Carrier | Calculated $(dm/dt)_t$ | Experimental $(dm/dt)_t$ | Exit Port Diameter |
|---|---|---|---|
| Hydrophilic Polymer | mg/day | mg/day | mm (mils) |
| 5,000,000 Molecular Weight | 79 | 77 | 7.6 (300) |
|  | 69 | *60 | 5.1 (200) |
| 7,500,000 Molecular Weight | 92 | 90 | 10.2 (400) |
|  | 79 | *66 | 7.6 (300) |

The data with an asterisk indicates the experimental value was prepared as follows: The dosage form was designed with one drug composition, comprising 79% or 6 g of lysocellin, and one expandable composition and one composition comprising calcium carbonate. The composition comprising calcium carbonate was used as a filler layer to conserve ionophore. The expandable composition was placed between the ionophore composition and the calcium carbonate composition. The ionophore composition faced the exit port and the calcium carbonate composition faced the density element. Within about three weeks after the experiment began the expandable composition began to occupy space between the wall and the calcium carbonate composition, thereby diverted some of the push energy away from the exit port, resulting in the indicated values.

The following examples are merely illustrative of the present invention, and they should not be construed as limiting the scope of the invention in any way, as these examples and other equivalents thereof will become more apparent to those skilled in the dispensing art in light of the present disclosure, the drawings and the accompanying claims.

EXAMPLE 1

A dosage form manufactured in she shape of a dispenser for the controlled delivery of lysocellin is made as follows: first, 85 g of lysocellin is passed through a 40 mesh screen. Then, 14.75 g of polyethylene oxide having a 5,000,000 molecular weight is passed through a 40 mesh screen. The just prepared lysocellin and polyethylene oxide are mixed thoroughly with 0.75 g of hydroxypropylmethyl cellulose having a 11,000 molecular weight to provide a homogeneous mix. Then, 30 ml of denatured, anhydrous ethanol is slowly added to the blending mixture, and all the ingredients mixed for an additional 2 to 3 minutes. The freshly prepared wet granulation is passed through a 20 mesh screen, allowed to dry at room temperature for 16 hours, and again passed through a 20 mesh screen to provide a drug composition.

Then, the drug composition is divided into two portions and compressed to make two different shaped tablets, or drug compositions. For one drug tablet, 7.06 g of the drug composition is compressed with a hydraulic press using a flat bottom tablet punch, and a deep-concave, top tablet punch. The other tablet is made by compressing 7.06 g of the drug composition using a flat bottom and flat top tablet punch.

Next, an expandable composition is prepared by passing separately through a 40 mesh screen the following ingredients: 84.7 g of sodium carboxymethylcellulose with a 700,000 molecular weight, 9.4 g of hydroxypropylcellulose with a 60,000 molecular weight, 4.7 g of sodium chloride and 1.0 g of ferric oxide. All of the above ingredients are thoroughly mixed to provide a homogeneous mass. Then, with continuous mixing, 40 ml of denatured alcohol is added slowly and the mixing continued for 2 to 3 minutes. The wet granulation is passed through a 20 mesh screen, dried at room temperature for 16 hours and again passed through a 20 mesh screen. Finally, 0.2 g of magnesium stearate is added to the granulation and the ingredients mixed in a rollermill for 3 to 4 minutes. The expandable composition is made into a tablet by compressing 5.2 g of the composition in a hydraulic press using a flat top and flat bottom tablet punch.

Next, a wall forming member designed and shaped like a cup is prepared as follows: first, 76 g of cellulose acetate butyrate having a butyryl content of 37% and 13% acetyl content, 15 g of polyethylene glycol having a 400 molecular weight and 9 g of triethyl citrate are mixed into a homogeneous mass. Then, 10.5 g of the mixture is injection molded to make a semipermeable walled cup with an average wall thickness of 65 mils.

The dispenser is assembled as follows: first, a 7.6 mm (300 mil) exit port is drilled through the concave end of the semipermeable cup. The first described drug tablet is then inserted into the semipermeable cup so that its convex top fits into the concave end of the cup. Then, the second described drug tablet is inserted into the cup so that it is flush against the flat end of the first inserted drug tablet. Next, the expandable tablet is inserted so that it is flush against the second drug tablet. Then, a 64 g iron densifier is inserted into the cup so that its flat end is against the expandable tablet. Finally, the wall is sealed as the open end is heated, pressed against the densifier and cooled to room temperature.

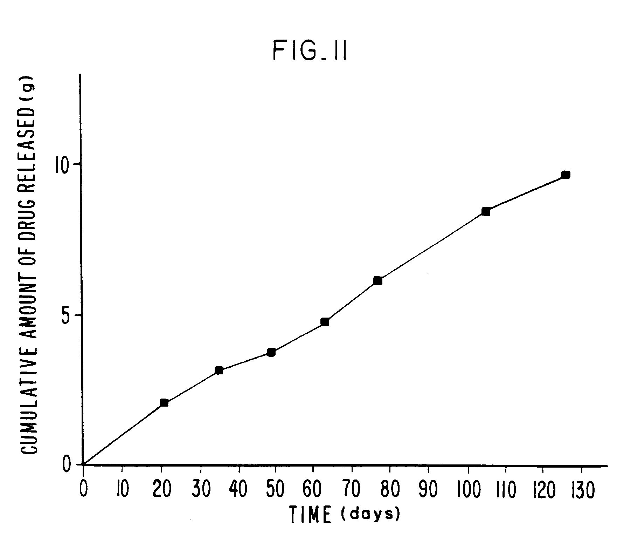

Dispensers prepared according to this example were placed into the fistulated cow's rumen. The dispensers were removed from the rumen at different time intervals to measure the amount of lysocellin released per unit time. Accompanying Figure 11 illustrates the controlled and continuous release of lysocellin at a rate of 85 mg/day for 126 days. In the figure, the squares denote the cumulative amount of lysocellin released (grms.) at different time (days) intervals, and the number of dispensers in the study where seven.

EXAMPLE 2

A dispenser sized and adapted for the controlled delivery of lysocellin is made according to the procedure set forth in Example 1, with all conditions as previously described, except that in this example the drug composition comprising the lysocellin is present as a single composition. The single composition weighs 14.12 g and comprises the polyethylene oxide having a 5,000,000 molecular weight, a hydroxypropylmethylcellulose having a 11,200 molecular weight, and the lysocellin ionophore.

EXAMPLE 3

A dispenser for administering two different carboxylic ionophores, monensin and lasoalocid, for increasing feed efficiency in ruminants is manufactured according to the procedure of Example 1. In this example, the dispenser comprises two ionophore compositions, with each composition comprising a different ionophore. A first composition nearest the exit port comprises monensin sodium, polyethylene oxide having a 5,000,000 molecular weight and hydroxypropylmethylcellulose having a 11,200 molecular weight. The second composition is in immediate contact with the first composition. The second composition comprises lasalocid, polyethylene oxide having a 3,000,000 molecular weight and hydroxypropylmethylcellulose having a 22,000 molecular weight. The rest of the dispenser is as described in Example 1. The use of two different ionophores delivered into the rumen operates to maintain maximum feed efficiency. The dispenser can be manufactured for administering the first ionophore composition for 50 to 60 days followed by delivering the second composition for 50 to 60 days.

EXAMPLE 4

A dispenser for administering two ionophores, narasin and tetronasin, simultaneously is manufactured as follows: first, 40 g of narsin and 40 g of tetronasin are blended into a homogeneous mass. Then, 14.75 g of a carboxyvinyl polymer of acrylic acid lightly cross-linked with polyallyl ether of sucrose and having a 3,000,000 molecular weight is mixed with the ionophores and the mixture passed through a 40 mesh stainless screen. Then, the freshly prepared mix is thoroughly blended with 1.25 g of hydroxypropylmethylcellulose possessing a 22,000 molecular weight and the blend screened as described. Next, all the screened ingredients are added to the bowl of a commercial blender and the ingredients dry blended for 15 to 20 minutes to produce a homogeneous blend. Then, a granulation fluid is prepared comprising 20 ml of ethanol and 20 ml of isopropyl alcohol and the granulating fluid added to the blending bowl as follows: first 10 ml is sprayed into the bowl with constant blending, then 30 ml of the granulation fluid is added slowly to the bowl and the wet mass blended for another 15 to 20 minutes. Then, the wet granules are passed through a 16 mesh screen and dried at room temperature for 24 hours. Then, 2 g of magnesium stearate is added to the dry granules, and the ingredients roll-mixed for 10 to 15 minutes on a standard two-roll mill.

Next, an expandable composition is prepared as follows: 85 g of polyethylene oxide having a 5,000,000 molecular weight is screened through a 40 mesh screen, then, 36 g of sodium chloride is passed through a 40 mesh screen and the ingredients added to a mixing bowl and blended for 10 to 15 minutes. Then, a granulation fluid is prepared by mixing 175 ml of methanol and 75 ml of isopropyl alcohol, and the granulation fluid added to the blending bowl in two steps. First, 75 ml of the granulation fluid is sprayed into the bowl with constant blending, then 175 ml of the granulation fluid is added slowly to the bowl and the wet blend mixed for 15 to 20 minutes to produce a homogeneous mass. Then, the wet blend is passed through a 16 mesh screen, spread on a stainless steel tray and dried at room temperature at 22.5°C for 16 hours. The dried blend is passed through a 16 mesh screen, then roll milled with 3 g of stearic acid on a two-roll mill for 20 to 30 minutes.

Next, a number of laminated drug cores are prepared by pressing the two compositions on a Stokes® layer press. The composition containing the ionophore is fed into the cavity of the press and compressed into a solid layer. Then, the composition comprising the expandable hydrophilic hydrogel and osmotic solute is fed into the cavity overlaying the compressed layer and pressed into a solid layer to form a two-layered ionophore expandable core.

Next, the core is placed in contacting arrangement with a density member inside an elongated mold. The expandable layer is in contact with the density member. The density member comprises frangible stainless steel having a density of 7.5 g/cc. The mold has an inside circumference slightly larger than the circumference of the core density member arrangement. The space between the inside of the mold and the arrangement is filled with a semipermeable composition. On drying the semipermeable composition forms a thin semipermeable wall around the laminated arrangement. The wall coated laminated member is removed from the mold and further coated with a semipermeable composition.

The semipermeable wall forming composition comprises 90 g of cellulose acetate butyrate having an butyryl content of 38%, and 10 g of poly(ethylene glycol) 4000 in a solvent comprising 2000 ml of methylene chloride and 700 ml of methanol. The laminated arrangement are coated with the semipermeable

13

wall forming composition until the desired wall surrounds the laminated arrangement. The coated laminated arrangement are then spread on a tray and the solvent evaporated in a circulating air oven at 40°C. After cooling to room temperature a full mouth passageway is cut through the semipermeable wall connecting the composition comprising the ionophores with the exterior of the dispenser. The dispenser will deliver the ionophores over a 130 day period of time.

EXAMPLE 5

A dispenser for delivering an ionophore to livestock is made by following the above examples. The dispenser of this example is 75 mm long and 25 mm in diameter. The dispenser comprises a pair of compositions in contacting arrangement, with each composition comprising 6.000 mg of an ionophore selected from the group consisting of lasolocid, lysocellin, septamycin, nigericin, dianemycin, monensin and salinomycin, 1,054 mg of polyethylene oxide possessing a 5,000,000 molecular weight, 162.50 mg of hydroxypropylmethylcellulose possessing a 11,200 molecular weight, and 125 mg of magnesium stearate. The dispenser comprises a single expandable layer in contact with the pair of ionophore composition. The expandable composition comprises 4,405 mg of sodium carboxymethyl cellulose having a 700,000 molecular weight, 490 mg of hydroxypropylcellulose, 245 mg of sodium chloride, 50 mg of ferric oxide, and 15 mg of magnesium stearate. The device contained a 64,000 mg iron densifier, a 10,500 mg cellulosic rate controlling wall, and a 10.2 mm (400 mil) diameter exit orifice.

EXAMPLE 6

A dispensing device for the controlled delivery of an ionophore into the digestive tract of an animal is manufactured as follows: first, 57 g of cellulose acetate having an acetyl content of 39.8% and 1.3 kg of cellulose acetate butyrate having a butyryl content of 38% are sized and then combined with 2.2 g of Citroflex® -2 triethylcitrate and 0.3 kg of polyethylene glycol 400 in the bowl of a large Hobart® mixer. After mixing for 20 minutes, the blended material is transferred to the feed hopper of a Van Dorn injection molder, which is equipped with a suitable mold to produce a 7.5 g cellulose cup having the approximate dimensions 6.3 cm in height x 2.1 cm in width and a wall thickness of 0.13 cm.

Next, 4.0 g of a hydrophilic expandable member comprising 70:30 ratio of sodium carboxymethylcellulose to sodium chloride, lubricated with 1% magnesium stearate is compressed using 10,000 lbs. of force in a Carver® laboratory press equipped with a tablet tool and then inserted into the cup.

Next, an ionophore composition comprising 10 g of an ionophore selected from the group consisting of lonomycin, lenotemycin, etheromycin, isolasalocid, laidomycin sodium salt, semduramicin, and alborixin potassium salt, 2.1 g of poly(ethylene oxide) having a 3,500,000 molecular weight, 0.325 g of hydroxypropylmethylcellulose having a 11,200 molecular weight and 0.5 g of magnesium stearate pressed into a solid table is inserted into the cup against the expandable member.

Then, an iron density element comprising four 1.27mm (50 mils) exit passageways, that possess the dual function of aiding in the retention of the dispenser in the rumen of an animal, and serving as a flow moderator through its multiplicity of passageways is inserted into the open end of the dispenser and seated against the ionophore composition. The protruding lip of the cup is heated until softened using a hot air gun capable of delivering 600°F air, and the lip crimped over the perimeter of the density element to provide the dispenser.

METHOD OF USING THE INVENTION

An embodiment of the invention pertains to a method for administering a beneficial ionophore at a controlled rate to the rumen of a ruminant. In carrying out the method, a dispenser is placed into a balling gun provided with an ejecting means, the gun inserted into the mouth of the animal beyond the base of the tongue, and the dispenser gently ejected by applying pressure to an ejection plunger in the gun, thereby sending the dispenser into the rumen. More specifically the method comprises the steps of: (A) admitting into an animal's rumen a dispenser comprising : (1) a wall comprising in at least a part a semipermeable polymer composition permeable to the passage of fluid and substantially impermeable to the passage of an ionophore, the wall surrounding; (2) an internal lumen comprising a layer comprising a beneficial ionophore and a pharmaceutically acceptable carrier for the ionophore; (3) a layer of an expandable hydrophilic polymeric composition in the lumen; (4) a dense member in the lumen for maintaining the dispenser in the rumen over a prolonged period of time, and at least one exit passageway in the wall that communicates with the composition comprising the ionophore and the carrier; (B) imbibing fluid through the semipermeable wall at a rate determined by the permeability of the wall and the osmotic pressure gradient across the wall, which fluid contacts the composition comprising the ionophore and forms a dispensable composition, and contacts the expandable composition causing the expandable compositions to expand and push against the ionophore composition; and (C) delivering the beneficial ionophore composition from the lumen by the expandable composition continually expanding against the ionophore composition causing the ionophore to be dispensed in a beneficially effective amount through the passageway at a controlled rate to the rumen over a prolonged period of time.

**Claims**

1. A dispensing device for delivering a beneficial ionophore to a fluid environment of use; the dispensing device comprising:-

   (a) wall means for surrounding and forming an internal compartment, said wall means being in at least a part semipermeable, being permeable to the passage of a fluid present in the environment of use;

   (b) a beneficial ionophore formulation in the compartment, said beneficial ionophore formulation forming with fluid that enters the compartment through the wall means in service a dispensable formulation;

   (c) means in the compartment for absorbing fluid from the environment of use through the wall means by osmosis and occupying an increasing amount of space in the compartment;

   (d) means in the compartment connecting the exterior of the device with the compartment for delivering the beneficial ionophore formulation to the environment of use over time;

   characterised in that said connecting means is a wide mouth passageway in the wall means having a surface area selected so as to maximise the release of the beneficial ionophore formulation by osmotic pumping and to minimise the release of the ionophore by diffusion, thereby to reduce the mechanical agitation dependant release to provide delivery of ionophore in a substantially controlled rate dose.

2. A dispensing device for delivering a beneficial ionophore to a fluid environment of use; the dispensing device comprising:-

   (a) wall means for surrounding and forming an internal compartment, said wall means being in at least a part semipermeable, being permeable to the passage of a fluid present in the environment of use;

   (b) a beneficial ionophore formulation in the compartment, said beneficial ionophore formulation forming with fluid that enters the compartment through the wall means in service a dispensable formulation;

   (c) means in the compartment for absorbing fluid from the environment of use through the wall means by osmosis and occupying an increasing amount of space in the compartment;

   (d) means in the compartment connecting the exterior of the device with the compartment for delivering the beneficial ionophore formulation to the environment of use over time;

   characterised in that the release rate of ionophore from the device is defined by the expression:-

   $$[dm/dt]_t = [dm/dt]_o + [dm/dt]_d$$

   in which $[dm/dt]_t$ is the total release rate of the ionophore, $[dm/dt]_o$ is the release rate of the beneficial agent due to the action of the osmotic pumping and $[dm/dt]_d$ is the release rate of the beneficial ionophore due to diffusion and in which $[dm/dt]_d$ is not greater than 33% of $[dm/dt]_t$.

3. A dispensing device as claimed in claim 2, characterised in that

   $$[dm/dt]_d = [D.Sep.Cs/h].L$$

   wherein

   D    is the diffusional coefficient of the beneficial ionophore in the fluid,

   Sep    is the surface area of the wide mouth passageway,

   h    is the thickness of the diffusion layer,

   Cs    is the solubility of beneficial ionophore formulation in the fluid, and

   L    is the percent ionophore present in the formulation;

   and wherein Sep is selected such that the rate of ionophore release due to diffusion is within the range 3% to 33% of the total ionophore release rate.

4. A dispensing device as claimed in claim 3 characterised in that the two major factors which contribute to $[dm/dt]_d$ are

(1) Sep, and

(2) Cs

the arrangement being such that for a given ionophore, Cs is constant such that $[dm/dt]_d$ is affected principally by Sep, and wherein Sep is selected to minimise $[dm/dt]_d$ and to maximise $[dm/dt]_o$.

5. A dispensing device as claimed in claim 2, claim 3 or claim 4 characterised in that

$$[dm/dt]_o = [k/h].Ap.\Delta\pi.\rho d.L$$

wherein

$\rho d$     is the ionophore formulation density,

L     is the percent ionophore in the formulation,

k     is the permeability of the wall means to the fluid at 40°C,

h     is the thickness of the wall means,

Ap     is the surface area of the fluid absorbing means in contact with the wall means,

$\Delta\pi$     is the fluid imbibation pressure,

and wherein in service $Ap.\Delta\pi$ remains substantially constant.

6. A dispenser as claimed in any preceding claim wherein the ionophore is lysocellin and the diameter of the wide mouth passageway is 50 mil (1.27 mm) to 400 mil (10.2 mm).

7. A dispenser as claimed in any of claims 1 to 5 wherein the ionophore is selected from lonomycin, lenotemycin, etheromycin, isolasalocid, laidlomycin, tetronasin, semduramicin and alborixin.

8. A dispenser as claimed in any of claims 1 to 5 wherein the ionophore is selected from lasalocid, septamycin, nigericin, dianemycin, monensin and salinomycin.

9. A dispenser as claimed in any preceding claim wherein the dispenser delivers the ionophore for 180 days.

10. A dispenser as claimed in any of claims 1 to 5 which further comprises another composition (16) in the lumen juxtaposed the first composition, the other composition comprising an ionophore (18) and a pharmaceutically acceptable carrier (20).

11. A dispenser as claimed in claim 10 wherein the ionophore (18) in the second composition (16) is lysocellin.

12. A dispenser as claimed in claim 10 or claim 11 wherein the first composition (14) and the second composition (16) comprise the same ionophore.

13. A dispenser as claimed in claim 10 or claim 11 wherein the first composition (14) and the second composition (16) comprise different ionophores one from the other.

14. A dispenser as claimed in claim 10, claim 11, claim 12 or claim 13 wherein the dispenser delivers ionophore for 200 days.

15. A dispenser as claimed in claim 10 wherein the ionophore (17) in the first composition (15) is selected from valinomycin, enniactin, monoactin, nonactin, dinactin, trinactin, virginiamycin, tetronasin, semduramicin, monensin, monensin sodium, nigericin, narasin and salinomycin.

16. A dispenser as claimed in claim 10 wherein the ionophore (17) in the first composition (15) is selected from isolasalocid, lasalocid, lysocellin, septamycin, laidomycin, lonomycin, lenotomycin, grisorixin, alborixin, etheromycin, azolmycin and sodium lysocellin.

17. A dispenser as claimed in claim 10, claim 15 or claim 16 wherein the ionophore (18) in the second composition (16) is selected from valinomycin, enniactin, monoactin, nonactin, dinactin, trinactin, virginiamycin, tetronasin, semduramicin, monensin, monensin sodium, nigericin, narasin and salinomycin.

18. A dispenser as claimed in claim 10, claim 15 or claim 16 wherein the ionophore (18) in the second composition (16) is selected from isolasalocid, lasalocid, lysocellin, septamycin, laidlomycin, lonomycin, lenotomycin, grisorixin, alborixin, etheromycin, azolomycin and sodium lysocellin.

## Patentansprüche

1. Abgabevorrichtung zur Verabreichung eines nützlichen Ionophoren an eine Fluid-Anwendungsumgebung, wobei die Abgabevorrichtung umfaßt:

   (a) eine mindestens zum Teil semipermeable Wandung, die eine Innenkammer umschließt und bildet, wobei die Wandung für den Durchtritt eines in der Anwendungsumgebung vorhandenen Fluids durchlässig ist,

   (b) eine nützliche Ionophorenzusammensetzung in der Kammer, die mit Fluid, das bei der Anwendung durch die Wandung in die Kammer eintritt, eine abgebbare Zusammensetzung bildet,

   (c) eine Vorrichtung in der Kammer, die Fluid aus der Anwendungsumgebung durch die Wandung hindurch durch Osmose aufnimmt und immer mehr Raum in der Kammer beansprucht,

   (d) eine Vorrichtung in der Kammer, die die äußere Umgebung der Vorrichtung mit der Kammer verbindet, um die nützliche Ionophorenzusammensetzung allmählich an die Anwendungsumgebung abzugeben,

   dadurch **gekennzeichnet**, daß die Verbindungsvorrichtung ein weiter Durchtrittskanal in der Wandung ist, dessen Oberfläche so gewählt ist, daß die Freisetzung der nützlichen Ionophorenzusammensetzung durch osmotisches Pumpen möglichst groß und die Ionophorenfreisetzung durch Diffusion möglichst gering ist, um so die durch mechanische Agitation bedingte Freisetzung zu vermindern und eine Verabreichung des Ionophoren in einer im wesentlichen gesteuerten Dosisrate zu gewährleisten.

2. Abgabevorrichtung zur Verabreichung eines nützlichen Ionophoren an eine Fluid-Anwendungsumgebung, wobei die Abgabevorrichtung umfaßt:

   (a) eine mindestens zum Teil semipermeable Wandung, die eine Innenkammer umschließt und bildet, wobei die Wandung für den Durchtritt eines in der Anwendungsumgebung vorhandenen Fluids durchlässig ist,

   (b) eine nützliche Ionophorenzusammensetzung in der Kammer, die mit Fluid, das bei der Anwendung durch die Wandung in die Kammer eintritt, eine abgebbare Zusammensetzung bildet,

   (c) eine Vorrichtung in der Kammer, die Fluid aus der Anwendungsumgebung durch die Wandung hindurch durch Osmose aufnimmt und immer mehr Raum in der Kammer beansprucht,

   (d) eine Vorrichtung in der Kammer, die die äußere Umgebung der Vorrichtung mit der Kammer verbindet, um die nützliche Ionophorenzusammensetzung allmählich an die Anwendungsumgebung abzugeben,

   dadurch **gekennzeichnet**, daß die Freisetzungsgeschwindigkeit des Ionophoren aus der Vorrichtung durch die folgende Formel ausgedrückt wird:

$$[dm/dt]_t = [dm/dt]_o + [dm/dt]_d$$

   wobei $[dm/dt]_t$ die Gesamtfreisetzungsgeschwindigkeit des Ionophoren, $[dm/dt]_o$ die Freisetzungsgeschwindigkeit des nützlichen Wirkstoffes aufgrund der osmotischen Pumpwirkung und $[dm/dt]_d$ die Freisetzungsgeschwindigkeit des nützlichen Ionophoren aufgrund der Diffusion ist, und wobei $[dm/dt]_d$ nicht größer ist als 33% von $[dm/dt]_t$.

3. Abgabevorrichtung nach Anspruch 2, dadurch **gekennzeichnet**, daß

$$[dm/dt]_d = [D.Sep.Cs/h].L$$

   wobei

   D      der Diffusionskoeffizient des nützlichen Ionophoren im Fluid,

   Sep    die Oberfläche des weiten Durchtrittskanals,

h die Dicke der Diffusionsschicht,

Cs die Löslichkeit der nützlichen Ionophorenzusammensetzung im Fluid und

L der Prozentanteil des in der Zusammensetzung enthaltenen Ionophoren ist,

und wobei Sep so gewählt ist, daß die Geschwindigkeit der Ionophorenfreisetzung aufgrund der Diffusion im Bereich von 3% bis 33% der Gesamtfreisetzungsgeschwindigkeit des Ionophoren liegt.

4. Abgabevorrichtung nach Anspruch 3, dadurch **gekennzeichnet**, daß die beiden Hauptfaktoren, die $[dm/dt]_d$ beeinflussen,

(1) Sep und

(2) Cs

sind, wobei gilt, daß Cs für einen bestimmten Ionophoren jeweils konstant ist, so daß $[dm/dt]_d$ hauptsächlich durch Sep beeinflußt wird, und wobei Sep so gewählt ist, daß $[dm/dt]_d$ möglichst klein und $[dm/dt]_o$ möglichst groß ist.

5. Abgabevorrichtung nach Anspruch 2, Anspruch 3 oder Anspruch 4, dadurch **gekennzeichnet**, daß

$$[dm/dt]_o = [k/h].Ap.\Delta\pi.\rho d.L$$

wobei

$\rho d$ die Dichte der Ionophorenzusammensetzung,

L der Prozentanteil des Ionophoren in der Zusammensetzung,

k die Permeabilität der Wandung für das Fluid bei 40 °C,

h die Dicke der Wandung,

Ap die Oberfläche der Fluidaufnahmevorrichtung, die mit der Wandung in Kontakt steht, und

$\Delta\pi$ der Fluidaufnahmedruck ist,

und wobei bei der Anwendung $Ap.\Delta\pi$ im wesentlichen konstant bleibt.

6. Abgabevorrichtung nach einem der vorhergehenden Ansprüche, wobei der Ionophor Lysocellin ist und der Durchmesser des weiten Durchtrittskanals 50 mil (1,27 mm) bis 400 mil (10,2 mm) beträgt.

7. Abgabevorrichtung nach einem der Ansprüche 1 bis 5, wobei der Ionophor ausgewählt ist aus Lonomycin, Lenotemycin, Etheromycin, Isolasalocid, Laidlomycin, Tetronasin, Semduramicin und Alborixin.

8. Abgabevorrichtung nach einem der Ansprüche 1 bis 5, wobei der Ionophor ausgewählt ist aus Lasalocid, Septamycin, Nigericin, Dianemycin, Monensin und Salinomycin.

9. Abgabevorrichtung nach einem der vorhergehenden Ansprüche, wobei die Abgabevorrichtung den Ionophoren über einen Zeitraum von 180 Tagen freisetzt.

10. Abgabevorrichtung nach einem der Ansprüche 1 bis 5, die ferner eine weitere Zusammensetzung (16) im Hohlraum enthält, die neben der ersten Zusammensetzung angeordnet ist und einen Ionophoren (18) und einen pharmazeutisch geeigneten Träger (20) enthält.

11. Abgabevorrichtung nach Anspruch 10, wobei der Ionophor (18) in der zweiten Zusammensetzung (16) Lysocellin ist.

12. Abgabevorrichtung nach Anspruch 10 oder Anspruch 11, wobei die erste Zusammensetzung (15) und die zweite Zusammensetzung (16) denselben Ionophoren enthalten.

13. Abgabevorrichtung nach Anspruch 10 oder Anspruch 11, wobei die erste Zusammensetzung (15) und die zweite Zusammensetzung (16) voneinander verschiedene Ionophoren enthalten.

14. Abgabevorrichtung nach Anspruch 10, Anspruch 11, Anspruch 12 oder Anspruch 13, wobei die Abgabevorrichtung den Ionophoren über einen Zeitraum von 200 Tagen hinweg abgibt.

15. Abgabevorrichtung nach Anspruch 10, wobei der Ionophor (17) in der ersten Zusammensetzung (15) ausgewählt ist aus Valinomycin, Enniactin, Monoactin, Nonactin, Dinactin, Trinactin, Virginiamycin, Tetronasin, Semduramicin, Monensin, Monensin-Natrium, Nigericin, Narasin und Salinomycin.

16. Abgabevorrichtung nach Anspruch 10, wobei der Ionophor (17) in der ersten Zusammensetzung (15) ausgewählt ist aus Isolasalocid, Lasalocid, Lysocellin, Septamycin, Laidomycin, Lonomycin, Lenotomycin, Grisorixin, Alborixin, Etheromycin, Azolmycin und Natriumlysocellin.

17. Abgabevorrichtung nach Anspruch 10, Anspruch 15 oder Anspruch 16, wobei der Ionophor (18) in der zweiten Zusammensetzung (16) ausgewählt ist aus Valinomycin, Enniactin, Monoactin, Nonactin, Dinactin, Trinactin, Virginiamycin, Tetronasin, Semduramicin, Monensin, Monensin-Natrium, Nigericin, Narasin und Salinomycin.

18. Abgabevorrichtung nach Anspruch 10, Anspruch 15 oder Anspruch 16, wobei der Ionophor (18) in der zweiten Zusammensetzung (16) ausgewählt ist aus Isolasalocid, Lasalocid, Lysocellin, Septamycin, Laidlomycin, Lonomycin, Lenotomycin, Grisorixin, Alborixin, Etheromycin, Azolomycin und Natriumlysocellin.

**Revendications**

1. Dispositif de libération destiné à libérer un ionophore bénéfique dans un milieu d'utilisation fluide; le dispositif de libération comprenant :

   (a) un moyen formant paroi entourant et formant un compartiment interne, ladite paroi étant au moins en partie semi-perméable, étant perméable au passage d'un fluide présent dans le milieu d'utilisation;

   (b) une formulation d'ionophore bénéfique dans le compartiment, ladite formulation d'ionophore bénéfique formant, avec le fluide qui pénètre dans le compartiment par le moyen formant paroi lors de son utilisation, une formulation pouvant être libérée;

   (c) un moyen dans le compartiment destiné à absorber le fluide du milieu d'utilisation par osmose à travers le moyen formant paroi et occupant une quantité d'espace croissante dans le compartiment;

   (d) un moyen dans le compartiment reliant l'extérieur du dispositif au compartiment afin de libérer la formulation d'ionophore bénéfique dans le milieu d'utilisation au cours du temps;

   caractérisé en ce que ledit moyen de connexion est un passage à large goulot dans le moyen formant paroi ayant une surface choisie de manière à maximiser la libération de la formulation d'ionophore bénéfique par pompage osmotique et à minimiser la libération de l'ionophore par diffusion, réduisant ainsi la libération due à l'agitation mécanique, afin d'assurer la libération de l'ionophore à un débit de dose essentiellement contrôlé.

2. Dispositif de libération destiné à libérer un ionophore bénéfique dans un milieu d'utilisation fluide; le dispositif de libération comprenant :

   (a) un moyen formant paroi entourant et formant un compartiment interne, ledit moyen formant paroi étant au moins en partie semi-perméable, étant perméable au passage d'un fluide présent dans le milieu d'utilisation;

   (b) une formulation d'ionophore bénéfique dans le compartiment, ladite formulation d'ionophore bénéfique formant avec le fluide qui pénètre dans le compartiment à travers le moyen formant paroi lors de son utilisation une formulation pouvant être libérée;

   (c) un moyen dans le compartiment destiné à absorber le fluide du milieu d'utilisation par osmose à travers le moyen formant paroi et occupant une quantité d'espace croissante dans le compartiment;

   (d) un moyen dans le compartiment reliant l'extérieur du dispositif au compartiment afin de libérer la formulation d'ionophore bénéfique dans le milieu d'utilisation au cours du temps;

   caractérisé en ce que la vitesse de libération de l'ionophore par le dispositif est définie par l'expression :

$$[dm/dt]_t = [dm/dt]_o + [dm/dt]_d$$

   dans laquelle $[dm/dt]_t$ est la vitesse de libération totale de l'ionophore, $[dm/dt]_o$ est la vitesse de libération de l'agent bénéfique due à l'action du pompage osmotique et $[dm/dt]_d$ est la vitesse de libération de l'ionophore bénéfique due à la diffusion, et dans laquelle $[dm/dt]_d$ ne représente pas plus de 33% de $[dm/dt]_t$.

EP 0 483 273 B1

**3.** Dispositif de libération selon la revendication 2, caractérisé en ce que

$$[dm/dt]_t = [D.Sep.Cs/h].L$$

dans laquelle

D est le coefficient de diffusion de l'ionophore bénéfique dans le fluide,
Sep est la surface du passage à large goulot,
h est l'épaisseur de la couche de diffusion,
Cs est la solubilité de la formulation d'ionophore bénéfique dans le fluide, et
L est le pourcentage d'ionophore présent dans la formulation;
et dans laquelle Sep est choisie de manière à ce que la vitesse de libération de l'ionophore due à la diffusion soit comprise dans la gamme de 3% à 33% de la vitesse de libération totale de l'ionophore.

**4.** Dispositif de libération selon la revendication 3, caractérisé en ce que les deux facteurs principaux qui contribuent à $[dm/dt]_d$ sont

(1) Sep et
(2) Cs

l'arrangement étant tel que pour un ionophore donné, Cs est constante de manière à ce que $[dm/dt]_d$ soit affecté principalement par Sep, et dans lequel Sep est choisi pour minimiser $[dm/dt]_d$ et pour maximiser $[dm/dt]_o$.

**5.** Dispositif de libération selon la revendication 2, 3 ou 4, caractérisé en ce que

$$[dm/dt]_o = [k/h].Ap.\Delta\pi.\rho d.L$$

dans laquelle

$\rho d$ est la densité de la formulation d'ionophore,
L est le pourcentage d'ionophore dans la formulation,
k est la perméabilité du moyen formant paroi vis-à-vis du fluide à 40°C,
h est l'épaisseur du moyen formant paroi,
Ap est l'aire du moyen d'absorption de fluide en contact avec le moyen formant paroi,
Dp est la pression d'imbibition de fluide,
et dans lequel, lors de son utilisation, Ap.Dp reste essentiellement constant.

**6.** Dispositif de libération selon l'une des revendications précédentes, dans lequel l'ionophore est la lysocelline et le diamètre du passage à large goulot est de 50 mil (1,27 mm) à 400 mil (10,2 mm).

**7.** Dispositif de libération selon l'une des revendications 1 à 5, dans lequel l'ionophore est choisi dans le groupe constitué par la lonomycine, lénotémycine, éthéromycine, isolasalocide, laidomycine, tétronasine, semduramycine et alborixine.

**8.** Dispositif de libération selon l'une des revendications 1 à 5, dans lequel l'ionophore est choisi dans le groupe constitué par la lasalocide, septamycine, nigéricine, dianémycine, monensine et salinomycine.

**9.** Dispositif de libération selon l'une des revendications précédentes, dans lequel le dispositif de libération libère l'ionophore pendant 180 jours.

**10.** Dispositif de libération selon l'une des revendications 1 à 5, qui comprend en outre une autre composition (16) dans la lumière juxtaposée à la première composition, l'autre composition comprenant un ionophore (18) et un véhicule (20) acceptable sur le plan pharmaceutique.

**11.** Dispositif de libération selon la revendication 10, dans lequel l'ionophore (18) dans la deuxième composition (16) est la lysocelline.

**12.** Dispositif de libération selon la revendication 10 ou 11, dans lequel la première composition (14) et la deuxième composition (16) comprennent le même ionophore.

20

**13.** Dispositif de libération selon la revendication 10 ou 11, dans lequel la première composition (14) et la deuxième composition (16) comprennent des ionophores différents.

**14.** Dispositif de libération selon la revendication 10, 11, 12 ou 13, dans lequel le dispositif de libération libère l'ionophore pendant 200 jours.

**15.** Dispositif de libération selon la revendication 10, dans lequel l'ionophore (17) dans la première composition (15) est choisi dans le groupe constitué par la valinomycine, enniactine, monoactine, nonactine, dinactine, trinactine, virginiamycine, tétronasine, semduramycine, monensine, monensine sodique, nigéricine, narasine et salinomycine.

**16.** Dispositif de libération selon la revendication 10, dans lequel l'ionophore (17) dans la première composition (15) est choisi dans le groupe constitué par la isolasalocide, lasalocide, lysocelline, septamycine, laidomycine, lonomycine, lenotomycine, grisorixine, alborixine, éthéromycine, azolmycine et lysocelline sodique.

**17.** Dispositif de libération selon la revendication 10, 15 ou 16, dans lequel l'ionophore (18) dans la deuxième composition (16) est choisi dans le groupe constitué par la valinomycine, enniactine, monoactine, nonactine, dinactine, trinactine, virginiamycine, tétronasine, semduramycine, monensine, monensine sodique, nigéricine, narasine et salinomycine.

**18.** Dispositif de libération selon la revendication 10, 15 ou 16, dans lequel l'ionophore (18) dans la deuxième composition (16) est choisi dans le groupe constitué par l'isolasalocide, lasalocide, lysocelline, septamycine, laidomycine, lonomycine, lenotomycine, grisorixine, alborixine, éthéromycine, azolmycine et lysocelline sodique.

FIG.1

FIG.2

FIG.3

FIG.4

FIG.5

FIG.6

FIG. 7

FIG. 8

## FIG.9

## FIG.10

FIG. II